# EUROPÄISCHE PATENTSCHRIFT

(11) **EP 1 198 395 B1**
(45) Veröffentlichungstag und Bekanntmachung des Hinweises auf die Patenterteilung: **16.02.2005**
(21) Anmeldenummer: 00943730.2
(22) Anmeldetag: 24.05.2000
(51) Int. Cl.: B65D 83/14, B21D 51/24, C22C 30/00, C22C 38/44, C22C 38/42

(54) **KANISTER ENTHALTEND EINE WIRKSTOFF-FORMULIERUNG MIT FLUORKOHLENWASSERSTOFF ALS TREIBMITTEL**
CANISTER COMPRISING A FORMULATION OF AN ACTIVE INGREDIENT AND A PROPELLANT FORMED OF A FLUOROHYDROCARBON
CONTENANT COMPRENANT UNE FORMULATION D'UN INGREDIENT ACTIF ET UN GAZ PROPULSEUR D'UN FLUOROHYDROCARBURE

(30) Priorität: 26.05.1999 DE 19924098
(43) Veröffentlichungstag der Anmeldung: 24.04.2002
(60) Teilanmeldung: 03027354.4
(73) Patentinhaber: Boehringer Ingelheim Pharma GmbH & Co.KG, 55218 Ingelheim am Rhein (DE)
(72) Erfinder: NAGEL, Juergen, D-55543 Bad Kreuznach (DE); HOELZ, Hubert, D-55413 Oberheimbach (DE); VEGA, Julio, César, AR-1429 Capital Federal Buenos Aires (AR); LOSTRITTO, Richard, Thomas, Gaithersburg, MD 20879-1844 (US)
(74) Vertreter: Kläs, Heinz-Gerd, Dr.
(86) Internationale Anmeldenummer: PCT/EP2000/004662
(87) Internationale Veröffentlichungsnummer: WO 2000/073170

(56) Entgegenhaltungen:
- DE-A- 4 112 303
- US-A- 4 888 516
- US-A- 5 340 838
- US-A- 5 494 636
- PATENT ABSTRACTS OF JAPAN vol. 014, no. 484 (P-1120), 22. Oktober 1990 (1990-10-22) -& JP 02 195543 A (SUMITOMO METAL MINING CO LTD), 2. August 1990 (1990-08-02)

## Beschreibung

Die vorliegende Erfindung betrifft einen korrosionsbeständigen Edelstahlkanister enthaltend eine treibmittelhaltige Aerosolformulierung mit einem Wirkstoff für die Inhalationstherapie.

### Hintergrund der Erfindung

In treibmittelbetriebenen Inhalatoren werden die Wirkstoffe zusammen mit dem Treibmittel in patronenähnlichen Kanistern gelagert. Diese Kanister bestehen in der Regel aus einem Aluminiumbehälter, der mit einer Ventiltasse aus Aluminium verschlossen ist, in die ein Ventil eingebettet ist. Ein solcher Kanister kann dann wie eine Patrone in den Inhalator eingeführt und verbleibt dort entweder permanent oder wird nach Gebrauch gegen eine neue Patrone ausgetauscht. Seitdem Anfang der 90er Jahre auf der Konferenz von Rio de Janeiro Fluorchlorkohlenwasserstoffe (FCKW, CFC) wegen ihrer Ozon-zerstörenden Eigenschaften weltweit geächtet wurden, wird als Alternative die Verwendung von Fluorkohlenwasserstoffen (FKW, HFA) für die Verwendung in treibmittelbetriebenen Inhalatoren angestrebt. Die zur Zeit vielversprechendsten Vertreter sind TG 134a und TG 227 (1,1,1,2,3,3,3-Heptafluorpropan). Dementsprechend müssen die bestehenden Applikationssysteme für die inhalative Therapie auf FCKW-freie Treibmittel umgestellt werden und neue Applikationssysteme und Wirkstoff- Formulierung entwickelt werden.

Überraschenderweise hat sich herausgestellt, daß Aluminiumkanister gegenüber Arzneistoff-Formulierungen mit Fluorkohlenwasserstoffen als Treibmittel nicht immer beständig sind, sondern in Abhängigkeit der Zusammensetzung der Formulierungen ein hohes Korrosionsrisiko aufweisen. Dies gilt insbesondere für Formulierungen, in denen Elektrolyte und/oder freie Ionen, besonders freie Halogenide vorliegen. In diesen Fällen wird das Aluminium angegriffen, so daß Aluminium als Mantelmaterial für die Kanister nicht verwendet werden kann. Ähnliche Unbeständigkeiten der Aluminiumkanister bei der Verwendung von Fluorkohlenwasserstoffen als Treibmittel werden beobachtet, wenn die Formulierungen saure oder basische Bestandteile enthalten, z.B. in Form der Wirkstoffe, der Zusatzstoffe, als Stabilisatoren, Surfactants, Geschmacksverstärker, Antioxidantien u.ä.

Ein Behälter aus nichtrostendem Stahl enthaltend ein nicht-elektrolythaltiges Dosieraerosol mit einem Fluorchlorkohlenwasserstoff als Treibmittel und einem pharmazeutischen Wirkstoff ist bekannt aus US-A-5 340 838.

### Beschreibung der Erfindung

Es ist eine der Aufgaben der vorliegenden Erfindung, einen Kanister für treibmittelbetriebene Inhalatoren zu schaffen, der korrosionsbeständig ist gegenüber elektrolyten-haltigen Wirkstoff-Formulierungen für die Inhalationstherapie mit einem Fluorkohlenwasserstoff als Treibmittel, der für die Verarbeitung und für den Gebrauch eine ausreichende Druck- und Bruchfestigkeit besitzt, der die Qualität der aufzubewahrenden Formulierungen gewährleistet und die anderen aus dem Stand der Technik bekannten Nachteile überwindet.

Eine weitere Aufgabe der Erfindung besteht darin, einen Kanister für treibmittelbetriebene Inhalatoren zu schaffen, dessen Behälter aus einem einzigen in sich homogenen Material besteht.

Überraschenderweise wurde gefunden, daß ein Kanister bestehend aus einem Behälter und einer Ventiltasse mit Ventil, bei dem zumindest der Behälter aus einer Legierung gemäß Anspruch 1 besteht und der Kanister bzw. Behälter eine elektrolyten-haltige Wirkstoff-Formulierung mit TG 134a und/oder TG 227 enthält, die erfindungsgemäße Aufgabe löst. Die Legierung enthält als Bestandteile Chrom (Cr), Nickel (Ni), Molybdän (Mo), Eisen (Fe) und Kohlenstoff (C). Die Legierung kann zusätzlich Kupfer (Cu), Mangan (Mn) und Silicium (Si) enthalten. Bevorzugt besteht der Behälter aus einer der unten beschriebenen Legierungen.

Die Erfindung betrifft ferner die Verwendung eines derartigen Behälters bzw. Kanisters, bestehend aus einem Behälter und einer Ventiltasse mit Ventil in treibmittelbetriebenen Dosieraerosolen (Inhalatoren) und ein Verfahren zur Herstellung derselben.

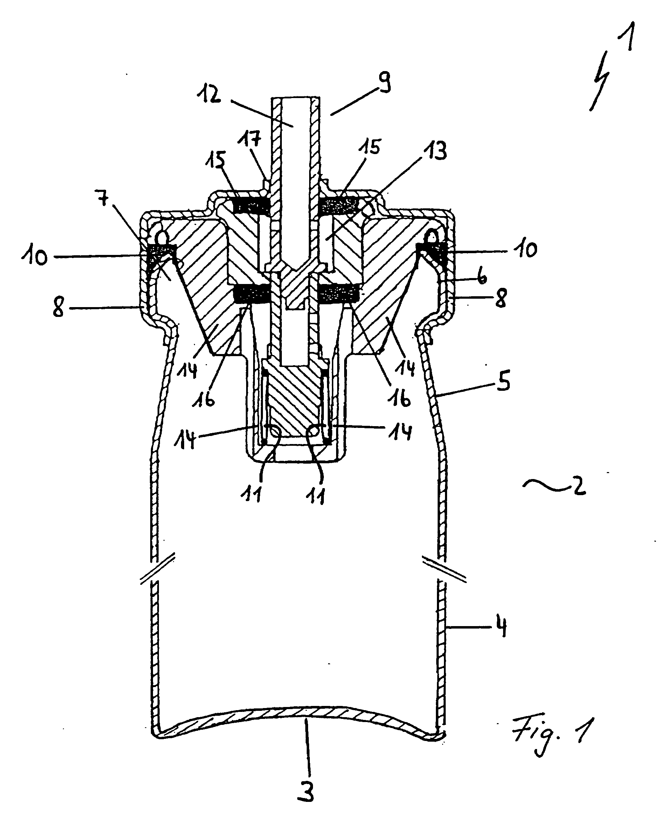

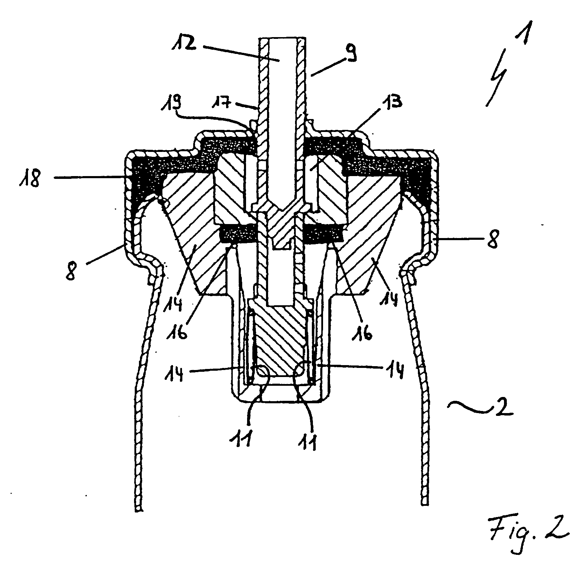

Im folgenden wird die Erfindung anhand der Figuren 1 und 2 näher besprochen.
Fig. 1 zeigt den Kanister bestehend aus Behälter (2), Ventiltasse (8) und das Ventil (9) im Querschnitt.
Fig. 2 zeigt eine weitere Ausführungsform der Ventiltasse (8) und des Ventils (9) im Querschnitt.

Figur 1 zeigt den erfindungsgemäßen Kanister (1) im Querschnitt. Der Kanister (1) besteht aus einem Behälter (2) zur Aufnahme der Arzneistoff-Formulierung und einer Ventiltasse (8) Figur 1 zeigt den erfindungsgemäßen Kanister (1) im Querschnitt. Der Kanister (1) besteht aus einem Behälter (2) zur Aufnahme der Arzneistoff-Formulierung und einer Ventiltasse (8) mit Ventil (9). Die Form und Dimension des Kanisters entspricht den aus dem Stand der Technik bekannten Aluminiumkanistern.

Der erfindungsgemäße Behälter (2) besteht aus einer Legierung mit einem Anteil an
Eisen von 40,0 - 53,0%,
Nickel 23,0 - 28,0%,
Chrom 19,0 - 23,0%,
Molybdän 4,0 - 5,0%,
Mangan 0,0 - 2,0%,
Kupfer 1,0 - 2,0%,
Silicium 0,0 - 1,0%,
Phosphor 0,0 - 0,045%,
Schwefel 0,0 - 0,035% und
Kohlenstoff 0,0 - 0,020%.
Bei dieser Legierung handelt es sich um eine Legierung gemäß der Werkstoffnummer 1.4539 der Stahl-Eisen-Liste des Vereins deutscher Eisenhüttenleute.

Eine bevorzugte Legierung dieser Art weist folgende Zusammensetzung auf:
Chrom 19,0 - 21,0%,
Nickel 24,0 - 26,0%,
Molybdän 4,0 - 5,0%,
Kupfer 1,0 - 2,0%,
Mangan bis zu 2,0%,
Silicium bis zu 0,5% und
Kohlenstoff bis zu 0,02%, wobei der restliche Bestandteil im wesentlichen Eisen ist.

In einer nahezu identischen Alternativlegierung ist der Molybdängehalt auf 4,5-5,0% eingeschränkt.

Die genannten Legierungen sind derart, daß sie gegenüber verschiedenen verflüssigten Fluorkohlenwasserstoffen wie TG 134a und TG 227 (1,1,1,2,3,3,3-Heptafluorpropan) korrosionsbeständig sind. Dazu zählen Treibgasformulierungen mit für die inhalative Therapie geeigneten Wirkstoffen, Surfactants, Co-Solventien, Stabilisatoren, Komplexbildnern, Geschmackskorrigentien, Antioxidantien, Salzen, Säuren, Basen oder Elektrolyten, wie beispielsweise Hydroxidionen, Cyanidionen und/oder Halogenidanionen wie Fluorid, Chlorid, Bromid oder Jodid.

Der Behälter (2) wird aus einem Mantel gebildet, der aus einer der oben beschriebenen Legierungen besteht. Der Behälter (2) weist vier verschiedene Zonen auf: den plan oder konkav nach innen gewölbten Boden (3), einen zylinderfömigen Bereich (4), der im oberen Drittel in den sich verjüngenden Hals (5) übergeht und schließlich in dem Wulst (6) endet, der die Öffnung (7) des Behälters umrandet.

Die Wandstärke des Behälters (2) beträgt in einer bevorzugten Ausführungsform zwischen 0,1 und 0,5 mm, bevorzugt zwischen 0,15 und 0,35 mm, ganz besonders bevorzugt ca. 0,19 bis 3,0 mm.

In einer bevorzugten Ausführungsform hält der Behälter (2) einem Berstdruck von mehr als 30000 hPa aus, bevorzugt mehr als 100000 hPa, ganz besonders bevorzugt mehr als 200000 hPa. Das Gewicht des Behälters (2) beträgt in einer bevorzugten Ausführungsform 5-15 g, in einer anderen 7 - 10 g und in wieder einer anderen 7,9 - 8,7 g. In einer gleichfalls bevorzugten Ausführungsform weist der Behälter (2) ein Volumen von 5 bis 50 ml auf. Andere Behälter weisen ein Volumen von 10 bis 20 ml und wieder andere von ca. 15 - 18 ml auf.

Im verschlossenen Zustand ist der Behälter (2) nach dem Befüllen mit der Arzneistoff-Formulierung und dem Treibmittel mit der Ventiltasse (8) dicht verschlossen.

In einer Ausführungsform besteht die Ventiltasse (8) ebenfalls aus korrosionsbeständigem Material. Bevorzugt handelt es sich dabei um die oben für den Behälter beschriebene Legierung und/oder Kunststoffmaterialien von geeigneter pharmazeutischer Qualität.

In einer anderen Ausführungsform besteht die Ventiltasse (8) aus Aluminium. In diesem Fall sind die Dichtung (10) und/oder das Ventil (9) derart ausgebildet, daß die Ventiltasse (8) selbst mit der im Inneren des Behälters befindlichen Flüssigkeit nicht in Berührung kommen kann.

Eine bevorzugte Ausführungsform der Ventiltasse (8) betrifft eine gemäß der GB 2324121, auf die hiermit in vollem Umfang Bezug genommen wird.

Im geschlossenen Zustand des Kanisters umcrimpt die Ventiltasse (8) den Behälter (2) an dessen Wulst (6). In bevorzugten Ausführungsformen dichtet eine Dichtung (10) die Ventiltasse (8) gegenüber dem Wulst (6) ab. Die Dichtung kann dabei ringförmig oder scheibenförmig sein. Bevorzugt ist sie scheibenförmig. Sie kann aus den aus dem Stand der Technik bekannten Materialien bestehen, die für die Verwendung von Arzneimittel-Formulierungen mit Fluorkohlenwasserstoffen als Treibmittel geeignet sind. Beispielsweise eigen sich hierfür Thermoplaste, Elastomere, Materialien wie Neopren, Isobutylen, Isopren, Butyl-Kautschuk, Buna-Kautschuk, Nitril-Kautschuk, Copolymere aus Ethylen und Propylen, Terpolymere aus Ethylen, Propylen und einem Dien, beispielsweise Butadien, oder fluorierte Polymere. Bevorzugtes Material sind Ethylen/Propylen-Dien-Terpolymere (EPDM).

Auf der zum Inneren des Behälters zugewandten Seite der Ventiltasse (8) ist ein Ventil (9) so ausgebildet, daß der Ventilstamm (12) durch die Ventiltasse (8) hindurch auf die andere Seite geht. Das Ventil (9) sitzt abdichtend in der zentralen Öffnung der Dichtung (10). Dichtung (10) und Ventil (9) zusammen dichten dabei die Ventiltasse (8) gegenüber dem Behälterinneren ab, so daß diese mit der Flüssigkeit in dem Behälter (2) nicht in Berührung kommen kann.

Das Ventil (9) ist derart aufgebaut, daß jedes Element, das mit der Flüssigkeit im Inneren des Behälters (2) in Berührung kommen kann, aus einem gegenüber dieser Flüssigkeit korrosionsbeständigem Material besteht. Zu diesen Elementen gehören beispielsweise die Feder oder Federn (11), der Ventilstamm (12), der durch die Öffnung (17) der Ventiltasse (8) von innen nach außen ragt, die Dosierkammer (13) und der Körper des Ventils (14). Die Feder (11) besteht aus Stahl, bevorzugt einem Edelstahl. Die weiteren Elemente des Ventils (9) können beispielsweise aus Stahl, der oben beschriebenen Legierung und/oder einem Kunststoff bestehen. Die Elemente (12), (13), und (14) bestehen bevorzugt aus einem Kunststoff, besonders bevorzugt aus einem Polyester, ganz besonders bevorzugt aus Polybutylenterephtalat.

Wie in Figur 1 dargestellt können eine oder mehrere weitere Dichtungen, z.B. die Dichtungen (15) und/oder (16), ausgebildet sein, die ein Entweichen von Flüssigkeit oder Gas aus dem Inneren des Behälters nach außen verhindern. Die Dichtung(en) kann (können) dabei auch so angeordnet sein, daß die Flüssigkeit im Inneren des Behälters außer mit der (den) Dichtung(en) selbst nur mit dem Behältermantel und dem Ventil in Kontakt kommt.

Die Dichtung (15) dichtet den optional vertikal beweglichen Ventilstamm an der Stelle ab, an der er die Ventiltasse (8) durchstößt. Die Dichtung (16) dichtet den Ventilstamm (12) im Inneren des Ventils gegenüber dem Ventilkörper (14) und/oder der Dosierkammer (13) ab. Damit verhindern die Dichtungen (15) und (16), daß eine Flüssigkeit oder ein Gas aus dem Inneren des Behälters entlang dem Außenmantel des Ventilstamms aus dem Kanister entweichen kann, bzw. über diesen Weg mit der Ventiltasse in Berührung kommt. Die Dichtungen (15) und (16) können aus dem gleichen Material wie die Dichtung (10) aufgebaut sein, bevorzugt einem Ethylen/Propylen-Dien-Terpolymer.

In einer Ausführungsform, bei der die Ventiltasse (8) nicht aus Aluminium, sondern aus einem der oben beschriebenen korrosionsbeständigen Materialien besteht, ist es nicht notwendig, daß die Dichtung (10) zusammen mit dem Ventil (9) die Ventiltasse vollständig gegenüber dem Behälterinneren isoliert. Daher ist es in diesem Fall auch nicht notwendig, daß die Dichtung (10) und das Ventil (9) einander abdichtend berühren. Gegebenenfalls besteht ein Spalt zwischen Dichtung (10) und Ventil (9). In einem solchen Fall sitzt die Dichtung (10) z. B. direkt auf der Unterseite der Ventiltasse (8) auf und dichtet den Rand der Ventiltasse (8) gegenüber dem Behälterwulst (6) ab. Die Dichtung (15) dichtet dann die Öffnung (17) in der Ventiltasse (8) gegenüber dem Inneren des Behälters ab.

Fig. 2 zeigt eine weitere Ausführungsform der Ventiltasse (8) mit eingebettetem Ventil (9). Diese Ausführungsform ist im großen und ganzen identisch mit der der Figur 1. Der wesentliche Unterschied besteht darin, daß die Dichtung (10) und die Dichtung (16) der Ausführungsform der Figur 2 zu der einen Dichtung (18) zusammengefaßt sind. Dichtung (18) ummantelt die Unterseite des Ventiltellers (8). Sie ist dergestalt, daß der Ventilkörper (14) in der Dichtung eingebettet vorliegt. Der Ventilstamm (12) passiert die Dichtung durch die Öffnung (19), die direkt unterhalb der Öffnung (17) der Ventiltasse (8) liegt. Die Öffnung (19) ist derart dimensioniert, daß sie den Ventilstamm (12) gegen die Ventiltasse (8) abdichtet. Das Dichtungsmaterial für die Dichtung (18) ist identisch mit dem für die Dichtung (10) beschriebenen.

Die Herstellung des erfindungsgemäße Behälters (2) geschieht in Analogie zu den nach aus dem Stand der Technik für Alukanister u.ä. bekannten Verfahren, nach denen der Mantel des Behälters aus einem Blech des entsprechenden Materials, bzw. der entsprechenden Legierung herausgestanzt wird.

Der Behälter (2) bzw. Kanister bestehend aus Behälter (2) und Ventiltasse (8) mit Ventil (9) ist zur Verwendung mit Fluorkohlenwasserstoff-haltigen Treibgasformulierungen geeignet. Treibgasformulierungen, die in Verbindung mit der Erfindung verwendet werden können, sind in der WO 94/13262 offenbart. Bevorzugt sind säurestabilisierte Treibgasformulierungen mit TG 134a und/oder 1,1,1,2,3,3,3-Heptafluorpropan (TG 227) als Treibgas, insbesondere solche die Ipatropiumbromid, Oxitropiumbromid, Albuterol, Tiotropiumbromid oder Fenoterol und als Wirkstoff enthalten.

Je nach Wirkstoff sind zum Stabilisieren anorganische und organische Säuren geeignet. Als Beispiele für anorganische Säuren werden neben Halogensäuren und weiteren Mineralsäuren genannt: Schwefelsäure, Salzsäure, Salpetersäure oder Phosphorsäure, als Beispiele organischer Säuren Ascorbinsäure oder Zitronensäure. Im Fall von Wirkstoffsalzen sind solche Säuren bevorzugt, deren Anion identisch ist mit dem des Wirkstoffsalzes. Für alle Wirkstoffe und Wirkstoffsalze ist generell Zitronensäure geeignet und auch am stärksten bevorzugt.

Der Gehalt an Säure ist derart, daß der pH-Wert der Formulierung zwischen 1,0 und 7,0, bevorzugt zwischen 2,0 und 5,0 und ganz besonders bevorzugt bei etwa 3,5 liegt. Im Fall von anorganischen Säuren befindet sich der bevorzugte Gehalt an Säure im Bereich von etwa 0,00002 bis 0,01 N. Im Fall von Ascorbinsäure befindet sich der bevorzugte Gehalt in etwa im Bereich von 0,0045 bis 5,0 mg/ml und im Fall der Zitronensäure im Bereich von 0,0039 bis 27,7 mg/ml.

Die Formulierungen können darüber hinaus Ethanol als Co-Solvens enthalten. Die bevorzugte Menge beträgt 1,0 bis 50,0 Gew.% der Formulierung,

Im folgenden sind beispielhaft bevorzugte Formulierungen, die in einer Kanister bzw. einem Behälter der oben beschriebenen Art gelagert werden können, aufgeführt:

| Beispiel 1 | |
|---|---|
| Ipatropiumbromid Monohydrat | 0,001- 2,5Gew.% |
| Ethanol, absolut | 0,001 - 50 Gew.% |
| TG 134a | 50,0 - 99,0 Gew.% |
| Anorganische Säure | 0,01 - 0,00002 Normal |
| Wasser | 0,0 - 5,0 Gew.%. |

| Beispiel 2 | |
|---|---|
| Ipatropiumbromid Monohydrat | 0,001 - 2,5Gew.% |
| Ethanol, absolut | 0,001 - 50 Gew.% |
| TG 134a | 50,0 - 99,0 Gew.% |
| Ascorbinsäure | 0,00015 - 5,0 mg/ml |
| Wasser (gereinigt) | 0,0 - 5,0 Gew.%. |

| Beispiel 3 | |
|---|---|
| Ipatropiumbromid Monohydrat | 0,0187 Gew.% |
| Ethanol, absolut | 15,0000 Gew.% |
| TG 134a | 84,4773 Gew.% |
| Zitronensäure | 0,0040 Gew.% |
| Wasser (gereinigt) | 0,5000 Gew.%. |
| Gesamt | 100,0000 Gew.% |

| Beispiel 4 | |
|---|---|
| Ipatropiumbromid Monohydrat | 0,0374 Gew.% |
| Ethanol, absolut | 15,0000 Gew.% |
| TG 134a | 84,4586 Gew.% |
| Zitronensäure | 0,0040 Gew.% |
| Wasser (gereinigt) | 0,5000 Gew.%. |
| Gesamt | 100,0000 Gew.% |

| Beispiel 5 | |
|---|---|
| Ipatropiumbromid Monohydrat | 0,0748 Gew.% |
| Ethanol, absolut | 15,0000 Gew.% |
| TG 134a | 84,4212 Gew.% |
| Zitronensäure | 0,0040 Gew.% |
| Wasser (gereinigt) | 0,5000 Gew.%. |
| Gesamt | 100,0000 Gew.% |

| Beispiel 6 | |
|---|---|
| Fenoterol Hydrobromid | 0,192 Gew.% |
| Ethanol, absolut | 30,000 Gew.% |
| TG 134a | 67,806 Gew.% |
| Zitronensäure | 0,002 Gew.% |
| Wasser (gereinigt) | 2,000 Gew.%. |
| Gesamt | 100,0000 Gew.% |

Eine Methode, die Kanister mit der entsprechenden Formulierung zu füllen, können beispielsweise die "Dual Stage Pressure Fill-Methode", die "Single Stage Cold Fill-Methode" oder die "Single Stage Pressure Fill-Methode" sein.

## Patentansprüche

1. Kanister (1) enthaltend eine Salz-, Säure-, Base- oder Elektrolyt-haltige Wirkstoff-Formulierung mit TG 134a und/oder TG 227 als Treibgas für die Inhalationstherapie, wobei der Kanister aus einem Behälter (2) und einer Ventiltasse (8) mit eingebettetem Ventil (9) besteht und der Behälter aus einer Legierung mit einem Anteil an Eisen von 40,0 - 53,0%, Nickel 23,0 - 28,0%, Chrom 19,0 - 23,0%, Molybdän 4,0 - 5,0%, Mangan 0,0 - 2,0%, Kupfer 1,0 - 2,0%, Silicium 0,0 - 1,0%, Phosphor 0,0 - 0,045%, Schwefel 0,0 - 0,035% und Kohlenstoff 0,0 - 0,020% gefertigt ist.

2. Kanister nach Anspruch 1, **dadurch gekennzeichnet, daß** der Behälter aus einer Legierung mit folgender Zusammensetzung besteht Chrom 19,0 - 21,0%, Nickel 24,0 - 26,0%, Molybdän 4,0 - 5,0%, Kupfer 1,0 - 2,0%, Mangan bis zu 2,0%, Silicium bis zu 0,5% und Kohlenstoff bis zu 0,02%, und mit Eisen als im wesentlichen restlichem Bestandteil.

3. Kanister nach einem der Ansprüche 1 bis 2, **dadurch gekennzeichnet, daß** die Ventiltasse (8) aus Aluminium besteht und mit einer Dichtung (10) und/oder (18) gegenüber dem Behälterinnenraum verschlossen ist.

4. Kanister nach einem der Ansprüche 1 bis 3, **dadurch gekennzeichnet, daß** das Ventil (9) eine oder mehrere Edelstahlfeder(n) (11), einen Ventilstamm (12), die Dosierkammer (13) und einen Ventilkörper (14) beinhaltet, wobei der Ventilstamm (12), die Dosierkammer (13) und/oder der Ventilkörper (14) aus Stahl, der Legierung nach einem der Ansprüche 1 bis 3 und/oder einem Kunststoff bestehen.

5. Kanister nach einem der Ansprüche 1 bis 4, **dadurch gekennzeichnet, daß** die Feder(n) (11) aus einem Edelstahl und der Ventilstamm (12), die Dosierkammer (13) und der Körper des Ventils (14) aus Polybutylenterephtalat bestehen.

6. Kanister nach einem der Ansprüche 1 bis 5, **dadurch gekennzeichnet, daß** der Ventilstamm (12) durch eine Dichtung (15) oder (18) gegenüber der Ventiltasse (8) abgedichtet ist.

7. Kanister nach einem der Ansprüche 1 bis 6, **dadurch gekennzeichnet, daß** die Dichtung (10) und/oder Dichtung (15) und/oder Dichtung (18) aus einem Ethylen/Propylen-Dien-Terpolymer besteht (bestehen).

8. Kanister nach einem der Ansprüche 1 bis 7, **dadurch gekennzeichnet, daß** die Ventiltasse (8) aus der gleichen Legierung wie der Behälter (2) besteht.

9. Kanister nach einem der Ansprüche 1 bis 8, **dadurch gekennzeichnet, daß** der Behälter einem Berstdruck von mehr als 30000 hPa, bevorzugt mehr als 100000 hPa standhält.

10. Kanister nach einem der Ansprüche 1 bis 9, **dadurch gekennzeichnet, daß** der Behälter einen Berstdruck von mehr als 200000 hPa standhält.

11. Kanister nach einem der Ansprüche 1 bis 10, **dadurch gekennzeichnet, daß** der Behälter eine Wandstärke von 0,1 bis 0,5 mm, bevorzugt 0,15 bis 0,35 mm, aufweist.

12. Kanister nach einem der Ansprüche 1 bis 10, **dadurch gekennzeichnet, daß** der Behälter eine Wandstärke von 0,19 bis 3,0 mm aufweist.

13. Kanister nach einem der Ansprüche 1 bis 11, **dadurch gekennzeichnet, daß** die Formulierung einen pH-Wert von 2,0 bis 5,0 hat.

14. Kanister nach einem der Ansprüche 1 bis 13, **dadurch gekennzeichnet, daß** die Wirkstoff-Formulierung Elektrolyten enthält, bevorzugt ausgewählt aus der Gruppe Hydroxidionen, Cyanidionen und/oder Halogenidanionen.

15. Kanister nach Anspruch 14, **dadurch gekennzeichnet, dass** die Elektrolyten ausgewählt sind aus Fluorid, Chlorid, Bromid oder Jodid.

16. Behälter enthaltend eine Salz-, Säure-, Base- oder Elektrolyt-haltige Wirkstoff-Formulierung mit TG 134a und/oder TG 227 als Treibgas für die Inhalationstherapie, **dadurch gekennzeichnet daß** der Behälter aus einer Legierung nach einem der Ansprüche 1 oder 2 besteht.

17. Behälter nach Anspruch 16, **dadurch gekennzeichnet, daß** der Behälter einen Berstdruck von mehr als 30000 hPa, bevorzugt mehr als 100000 hPa standhält.

18. Behälter nach Anspruch 16, **dadurch gekennzeichnet, daß** der Behälter einen Berstdruck von mehr als 200000 hPa standhält.

19. Behälter nach einem der Ansprüche 16 bis 18, **dadurch gekennzeichnet, daß** der Behälter eine Wandstärke von 0,1 bis 0,5 mm, bevorzugt 0,15 bis 0,35 mm, aufweist.

20. Behälter nach einem der Ansprüche 16 bis 18, **dadurch gekennzeichnet, daß** der Behälter eine Wandstärke von 0,19 bis 3,0 mm aufweist.

21. Behälter nach einem der Ansprüche 16 bis 20, **dadurch gekennzeichnet, daß** die Formulierung einen pH-Wert von 2,0 bis 5,0 hat.

22. Behälter nach einem der Ansprüche 16 bis 21, **dadurch gekennzeichnet, daß** die Wirkstoff-Formulierung Elektrolyten enthält, bevorzugt ausgewählt aus der Gruppe Hydroxidionen, Cyanidionen und/oder Halogenidanionen.

23. Behälter nach Anspruch 22, **dadurch gekennzeichnet, dass** die Elektrolyten ausgewählt sind aus Fluorid, Chlorid, Bromid oder Jodid.

24. Verwendung eines Kanisters nach den Ansprüchen 1 bis 15 oder eines Behälters nach einem der Ansprüche 16 bis 23 in einem Inhalator und/oder zur Lagerung der Wirkstoffformulierungen, **dadurch gekennzeichnet, daß** die Wirkstoffformulierung Ethanol als Co-Solvens und Ipatropiumbromid, Oxitropiumbromid, Tiotropiumbromid, Albuterol oder Fenoterol als Wirkstoff enthält.

25. Verwendung Anspruch 24, **dadurch gekennzeichnet, daß** die Wirkstoffformulierung Ethanol und Ipatropiumbromid, Oxitropiumbromid oder Tiotropiumbromid als Wirkstoff enthält.

26. Verwendung nach Anspruch 24 oder 25, **dadurch gekennzeichnet, daß** die Formulierung Zitronensäure enthält.

27. Verwendung nach Anspruch 24 oder 25, **dadurch gekennzeichnet, daß** die Formulierung eine Mineralsäure enthält.

28. Verwendung nach Anspruch 24 oder 25, **dadurch gekennzeichnet, daß** die Formulierung Salzsäure enthält.

## Claims

1. Canister (1) containing an active substance formulation which contains salt, acid, base or electrolyte with TG 134a and/or TG 227 as propellant gas for inhalation therapy, the canister consisting of a container (2) and a valve cup (8) with valve (9) embedded therein, and the container being made of an alloy containing 40.0 - 53.0% iron, 23.0 - 28.0% nickel, 19.0 - 23.0% chromium, 4.0 - 5.0% molybdenum, 0.0 - 2.0% manganese, 1.0 - 2.0% copper, 0.0 - 1.0% silicon, 0.0 - 0.045% phosphorus, 0.0 - 0.035% sulphur and 0.0 - 0.020% carbon.

2. Canister according to claim 1, **characterised in that** the container consists of an alloy having the following composition: chromium 19.0 - 21.0%, nickel 24.0 - 26.0%, molybdenum 4.0 - 5.0%, copper 1.0 - 2.0%, manganese up to 2.0%, silicon up to 0.5% and carbon up to 0.02%, with iron substantially constituting the remaining ingredient.

3. Canister according to one of claims 1 to 2, **characterised in that** the valve cup (8) consists of aluminium and is sealed with a gasket (10) and/or (18) relative to the interior of the container.

4. Canister according to one of claims 1 to 3, **characterised in that** the valve (9) contains one or more stainless steel springs (11), a valve stem (12), the metering chamber (13) and a valve body (14), the valve stem (12), the metering chamber (13) and/or the valve body (14) being made of steel, the alloy according to one of claims 1 to 3 and/or a plastic.

5. Canister according to one of claims 1 to 4, **characterised in that** the spring(s) (11) consist(s) of a stainless steel and the valve stem (12), the metering chamber (13) and the body of the valve (14) consist of polybutylene terephthalate.

6. Canister according to one of claims 1 to 5, **characterised in that** the valve stem (12) is sealed off from the valve cup (8) by a gasket (15) or (18).

7. Canister according to one of claims 1 to 6, **characterised in that** the gasket (10) and/or gasket (15) and/or gasket (18) consist or consists of an ethylene/propylene/diene terpolymer.

8. Canister according to one of claims 1 to 7, **characterised in that** the valve cup (8) consists of the same alloy as the container (2).

9. Canister according to one of claims 1 to 8, **characterised in that** the container withstands a bursting pressure of more than 30,000 hPa, preferably more than 100,000 hPa.

10. Canister according to one of claims 1 to 9, **characterised in that** the container withstands a bursting pressure of more than 200,000 hPa.

11. Canister according to one of claims 1 to 10, **characterised in that** the container has a wall thickness of 0.1 to 0.5 mm, preferably 0.15 to 0.35 mm.

12. Canister according to one of claims 1 to 10, **characterised in that** the container has a wall thickness of 0.19 to 3.0 mm.

13. Canister according to one of claims 1 to 11, **characterised in that** the formulation has a pH of 2.0 to 5.0.

14. Canister according to one of claims 1 to 13, **characterised in that** the active substance formulation contains electrolytes which are preferably selected from among the hydroxide ions, cyanide ions and/or halide anions.

15. Canister according to claim 14, **characterised in that** the electrolytes are selected from among fluoride, chloride, bromide or iodide.

16. Container containing an active substance formulation which contains salt, acid, base or electrolyte with TG 134a and/or TG 227 as the propellent gas for inhalation therapy, **characterised in that** the container consists of an alloy according to one of claims 1 and 2.

17. Container according to claim 16, **characterised in that** the container withstands a bursting pressure of more than 30,000 hPa, preferably more than 100,000 hPa.

18. Container according to claim 16, **characterised in that** the container withstands a bursting pressure of more than 200,000 hPa.

19. Container according to one of claims 16 to 18, **characterised in that** the container has a wall thickness of 0.1 to 0.5 mm, preferably 0.15 to 0.35 mm.

20. Container according to one of claims 16 to 18, **characterised in that** the container has a wall thickness of 0.19 to 3.0 mm.

21. Container according to one of claims 16 to 20, **characterised in that** the formulation has a pH of 2.0 to 5.0.

22. Container according to one of claims 16 to 21, **characterised in that** the active substance formulation contains electrolytes which are preferably selected from among the hydroxide ions, cyanide ions and/or halide anions.

23. Container according to claim 22, **characterised in that** the electrolytes are selected from among fluoride, chloride, bromide or iodide.

24. Use of a canister according to claims 1 to 15 or a container according to one of claims 16 to 23 in an inhaler and/or for storing active substance formulations, **characterised in** the active substance formulation contains ethanol as cosolvent and ipatropium bromide, oxitropium bromide, tiotropium bromide, albuterol or fenoterol as active substance.

25. Use according to claim 24, **characterised in that** the active substance formulation contains ethanol and ipatropium bromide, oxitropium bromide or tiotropium bromide as active substance.

26. Use according to claim 24 or 25, **characterised in that** the formulation contains citric acid.

27. Use according to claim 24 or 25, **characterised in that** the formulation contains a mineral acid.

28. Use according to claim 24 or 25, **characterised in that** the formulation contains hydrochloric acid.

## Revendications

1. Récipient (1) contenant une formulation de principe actif contenant un sel, un acide, une base ou un électrolyte avec du TG 134a et/ou du TG 227 comme gaz propulseur pour la thérapie par inhalation, le récipient consistant en un conteneur (2) et un logement de soupape (8) avec une soupape encastrée (9), et le conteneur est en un alliage avec une part de fer de 40,0 - 53,0 %, de nickel 23,0 - 28,0 %, de chrome 19,0 - 23,0 %, de molybdène 4,0 - 5,0 %, de manganèse 0,0 - 2,0 %, de cuivre 1,0 - 2,0 %, de silicium 0,0 - 1,0 %, de phosphore 0,0 - 0,045 %, de soufre 0,0 - 0,035 % et de carbone 0,0 - 0,020 %.

2. Récipient selon la revendication 1,
**caractérisé en ce que** le conteneur est en un alliage avec la composition suivante chrome 19,0 - 21,0 %, nickel 24,0 - 26,0 %, molybdène 4,0 - 5,0 %, cuivre 1,0 - 2,0 %, manganèse jusqu'à 2,0 %, silicium jusqu'à 0,5 % et carbone jusqu'à 0,02 %, et avec du fer en tant que composant résiduel principal.

3. Récipient selon l'une des revendications 1 à 2, **caractérisé en ce que** le logement de soupape (8) est en aluminium et est fermé avec un joint (10) et/ou (18) par rapport à l'espace interne du conteneur.

4. Récipient selon l'une des revendications 1 à 3, **caractérisé en ce que** la soupape (9) contient un ou plusieurs ressorts (11) en acier inoxydable, une tige de soupape (12), la chambre de dosage (13) et un corps de soupape (14), la tige de soupape (12), la chambre de dosage (13) et/ou le corps de soupape (14) étant en acier, en l'alliage selon l'une des revendications 1 à 3 et/ou en matière plastique.

5. Récipient selon l'une des revendications 1 à 4, **caractérisé en ce que** le ou les ressorts (11) sont en un acier inoxydable, et la tige de soupape (12), la chambre de dosage (13) et le corps de la soupape (14) en polybutylène téréphtalate.

6. Récipient selon l'une des revendications 1 à 5, **caractérisé en ce que** l'étanchéité de la tige de soupape (12) est assurée par un joint d'étanchéité (15) ou (18) vis-à-vis du logement de soupape (8).

7. Récipient selon l'une des revendications 1 à 6, **caractérisé en ce que** le joint (10) et/ou le joint (15) et/ou le joint (18) est/sont en un terpolymère éthylène/propylène-diène.

8. Récipient selon l'une des revendications 1 à 7, **caractérisé en ce que** le logement de soupape (8) est dans le même alliage que le conteneur (2).

9. Récipient selon l'une des revendications 1 à 8, **caractérisé en ce que** le conteneur résiste à une pression d'éclatement supérieure à 30 000 hPa, de préférence supérieure à 100 000 hPa.

10. Récipient selon l'une des revendications 1 à 9, **caractérisé en ce que** le conteneur résiste à une pression d'éclatement supérieure à 200 000 hPa.

11. Récipient selon l'une des revendications 1 à 10,
**caractérisé en ce que** le conteneur présente une épaisseur de paroi de 0,1 à 0,5 mm, de préférence 0,15 à 0,35 mm.

12. Récipient selon l'une des revendications 1 à 10,
**caractérisé en ce que** le conteneur présente une épaisseur de paroi de 0,19 à 3,0 mm.

13. Récipient selon l'une des revendications 1 à 11,
**caractérisé en ce que** la formulation présente une valeur de pH de 2,0 à 5,0.

14. Récipient selon l'une des revendications 1 à 13,
**caractérisé en ce que** la formulation de principe actif contient des électrolytes, de préférence choisis dans le groupe des ions hydroxyde, ions cyanure et/ou ions halogénure.

15. Récipient selon la revendication 14, **caractérisé en ce que** les électrolytes sont choisis parmi fluorure, chlorure, bromure ou iodure.

16. Conteneur contenant une formulation de principe actif contenant un sel, un acide, une base ou un électrolyte avec du TG 134a et/ou TG 227 comme gaz propulseur pour la thérapie par inhalation,
**caractérisé en ce que** le conteneur consiste est un alliage selon l'une des revendications 1 ou 2.

17. Conteneur selon la revendication 16, **caractérisé en ce que** le conteneur résiste à une pression d'éclatement supérieure à 30 000 hPa, de préférence supérieure à 100 000 hPa.

18. Conteneur selon la revendication 16, **caractérisé en ce que** le conteneur résiste à une pression d'éclatement supérieure à 200 000 hPa.

19. Conteneur selon l'une des revendications 16 à 18,
**caractérisé en ce que** le conteneur présente une épaisseur de paroi de 0,1 à 0,5 mm, de préférence 0,15 à 0,35 mm.

20. Conteneur selon l'une des revendications 16 à 18,
**caractérisé en ce que** le conteneur présente une épaisseur de paroi de 0,19 à 3,0 mm.

21. Conteneur selon l'une des revendications 16 à 20,
**caractérisé en ce que** la formulation a une valeur de pH de 2,0 à 5,0.

22. Conteneur selon l'une des revendications 16 à 21,
**caractérisé en ce que** la formulation du principe actif contient des électrolytes, de préférence choisis dans le groupe des ions hydroxyde, ions cyanure et/ou ions halogénure.

23. Conteneur selon la revendication 22, **caractérisé en ce que** les électrolytes sont choisis parmi fluorure, chlorure, bromure ou iodure.

24. Utilisation d'un récipient selon les revendications 1 à 15, ou d'un conteneur selon l'une des revendications 16 à 23 dans un inhalateur et/ou pour le stockage de formulations de principe actif,
**caractérisée en ce que** la formulation de principe actif contient de l'éthanol en tant que co-solvant et du bromure d'ipatropium, du bromure d'oxitropium, du bromure de thiotropium, de l'albutérol ou du fénotérol en tant que principe actif.

25. Utilisation selon la revendication 24, **caractérisée en ce que** la formulation du principe actif contient de l'éthanol et du bromure d'ipatropium, du bromure d'oxitropium ou du bromure de tiotropium en tant que principe actif.

26. Utilisation selon la revendication 24 ou 25, **caractérisée en ce que** la formulation contient de l'acide citrique.

27. Utilisation selon la revendication 24 ou 25, **caractérisée en ce que** la formulation contient un acide minéral.

28. Utilisation selon la revendication 24 ou 25, **caractérisée en ce que** la formulation contient de l'acide chlorhydrique.
